# EUROPEAN PATENT APPLICATION

(11) **EP 1 198 986 A1**
(43) Date of publication of application: **24.04.2002**
(21) Application number: 01922012.8
(22) Date of filing: 23.04.2001
(51) Int. Cl.: A01K 13/00, A01K 29/00, H04M 11/00

(54) **DOMESTIC ANIMAL DELIVERY WAIT MANAGING SYSTEM**

(30) Priority: 21.04.2000 JP 2000121379
(71) Applicant: Harada Electronics Co., Ltd, Sapporo-shi, Hokkaido 060-0042 (JP)
(72) Inventor: HARADA, Masahide, HARADA ELECTRONICS CO., LTD., Sapporo-shi, Hokkaido 060-0042 (JP)
(74) Representative: Lamb, Martin John Carstairs
(86) International application number: JP0103466
(87) International publication number: WO0180630

(57) **Abstract**

An object of the present invention is to lighten an observation work on livestock by an observer such as a specialized veterinarian, to reduce the expenses of an administrator of the livestock such as a stock farmer, and to save the labor of the administrator by providing a system of standby management for livestock birth which advantageously solves problems in the conventional observation system on the livestock being about to calve.

A system of standby management for livestock birth comprises: one or more livestock biological data measurement means 1 for measuring changes in physical conditions due to the birth and outputting them as measurement data; a data relay means 2 for putting the measurement data from the livestock biological data measurement means on a communication line; a data display means 3 for receiving and displaying the measurement data transmitted via the communication line; and an information means 4 for informing an administrator of the livestock about the birth when it is judged that the livestock has the birth sign or the livestock is calving based on the results displayed on the data display means.

## Description

### Technical Field

The present invention relates to a system of standby management for livestock birth which lighten a burden of a observer for a birth sign and a administrator of livestock, such as a livestock breeder, by remotely monitoring a change in a physical condition of livestock bred by a stock farmer or the like due to the birth and informing the administrator about the birth when it is judged that the livestock has the birth sign or the livestock is calving.

### Background Art

Conventionally, when livestock is about to calve, a person having some expert knowledge or experience is in constant attendance on the livestock and measures factors for judging changes in physical conditions due to the birth such as a body temperature, a respiration rate and a heart rate. An expression of the birth sign of the livestock is watched based on the measured data. When the expression of the birth sign is detected, the person informs a livestock breeder or the like about such a sign to encourage to standby for the birth.

According to the above conventional method, however, it cannot be recognized when the birth sign of the livestock is expressed unless the person having some expert knowledge or experience is in constant attendance on the livestock and measures the physical conditions of the livestock due to the birth. Therefore, when the due date of the livestock is close, the livestock sometimes should be watched night and day for a long time, e.g., for two or three days in the longest case. In such a case, there is a problem that the attendant of the above livestock may expend enormous energies, or it drives up the costs of birth management to be paid by the administrator such a stock farmer (especially, an owner of the livestock).

Furthermore, the expert knowledge and the experience are required to judge the condition of the livestock such as the expression of the birth sign of the livestock. Therefore, the number of the above attendants of the livestock is limited, so that it is difficult to take a human-wave tactic.

As the number of livestock bred by the stock farmer increases, the number of the pregnant livestock would be naturally increased. Thus, two or more livestock may calve at a time. In such a case, there is a problem that the person who watches the expression of the birth sign may be forced to do extremely heavy work.

### Disclosure of the Invention

An object of the present invention is to lighten the observation work on the livestock by the observer such as a specialized veterinarian, to reduce the expenses of the administrator of the livestock such as the stock farmer, and to save the labor of the administrator by providing a system of standby management for livestock birth which advantageously solves the problems in the conventional observation system on the livestock being about to calve with a view to the above points.

A system of standby management for livestock birth according to the present invention comprises: one or more livestock biological data measurement means for measuring changes in physical conditions due to the birth and outputting them as measurement data; a data relay means for putting the measurement data from the livestock biological data measurement means on a communication line; a data display means for receiving and displaying the measurement data transmitted via the communication line; and an information means for informing an administrator of the livestock about the birth when it is judged that the livestock has the birth sign or the livestock is calving based on the results displayed on the data display means.

In the system of standby management for livestock birth according to the invention, one or more livestock biological data measurement means such as a body temperature sensor equipped on a part of the body of the livestock measures changes in physical conditions (e.g., a body temperature) of the livestock due to the birth and outputs a measured value as measurement data. The data relay means puts the measurement data from the livestock biological data measurement means on the communication line, and the data display means receives the measurement data transmitted through the communication line to display it thereon. Subsequently, when an observer having some expert knowledge or experience, such as a specialized veterinarian, judges that the livestock has a birth sign or the livestock is calving based on the results displayed on the measurement data display means, the information means informs the administrator of the livestock about the birth information including the fact that the livestock has the birth sign or the livestock is calving and the information to specify the livestock.

According to the system of standby management for livestock birth of the present invention, therefore, a sensor for measuring the changes in physical conditions of the livestock due to the birth may be pre-attached on the livestock being about to calve (e.g., a springing cow) to allow a continuous representation of the measurement data of the changes in the physical conditions of the livestock due to the birth transmitted from the sensor on a display means (e.g., a data display device) equipped, for example, on a livestock management center away from the stock farmer or the like where the livestock is bred. Therefore, an observation of the livestock and a measurement on the changes in physical conditions of the livestock due to the birth by attending on the livestock, which should be conventionally performed to judge whether the livestock has a birth sign or whether the livestock is calving, can be omitted. Thus, it may lighten a burden of the observer such as the specialized veterinarian and reduce expenses for birth management of the administrator of the stock farmer or the like (especially, the owner of the livestock).

Furthermore, in the case where a stock farmer or the like breeds many livestock, two or more livestock may calve at a time. Even in such a case, as described above, there is no need to perform the measurement work in which the observer is directly in attendance with the livestock. The observer may only make a judgment based on the data displayed on the data display means such as a display. Thus, the above livestock management center or the like is allowed to extensively and simultaneously observe and manage the data of physical conditions of the livestock such as the respective birth signs of many livestock as a unit. In addition, it can be performed by a small number of the observers with reduced burden of each person.

On the other hand, the system of standby management for livestock birth according to the present invention may comprise: one or more livestock biological data measurement means for measuring changes in physical conditions of the livestock due to the birth and outputting them as measurement data; a data relay means for putting the measurement data from the livestock biological data measurement means on a communication line; a livestock biological data judgment means for making a judgment whether the livestock has a birth sign or whether the livestock is calving based on the received measurement data transmitted via the communication line; and an information means for informing an administrator of the livestock about the birth information when it is judged that the livestock has the birth sign or the livestock is calving based on the results displayed on the data display means.

In the system of standby management for livestock birth according to the present invention, one or more livestock biological data measurement means, such as a body temperature sensor equipped on a part of the body of the livestock, measures the changes in physical conditions of the livestock due to the birth and outputs a measured value as measurement data. The data relay means puts the measurement data on the communication line, and then the livestock biological data judgment means makes a judgment whether the livestock has a birth sign or whether the livestock is calving based on the received measurement data transmitted via the measurement line. When it is judged that the livestock has a birth sign or the livestock is calving, the information means informs the administrator of the livestock about birth information including the fact that the livestock has a birth sign or the livestock is calving and the information to specify the livestock. The birth information may also include the above biological data.

According to the system of standby management for livestock birth, therefore, a sensor for measuring the changes in physical conditions of the livestock due to the birth may be pre-attached on the livestock being about to calve (e.g., a springing cow) to send the measurement data of the changes in physical conditions of the livestock transmitted from the sensor, for example to the livestock biological data judgment means (e.g., an automatic judgment device) equipped on a livestock management center away from the stock farmer or the like where the livestock is bred. In the livestock biological data judgment means, the judgment can be made, for example, by executing a program by a personal computer or the like, where an algorism is incorporated in a program to judge whether the livestock has a birth sign or whether the livestock is calving, followed by performing a predetermined process. When the livestock is judged to have a birth sign or to be calving, a signal is automatically output to the information means. Then, the information means informs the administrator of the livestock about it through a facsimile machine or the like.

Accordingly, the livestock biological data judgment means automatically makes a judgment whether the livestock has a birth sign or whether the livestock is calving, and then the information means can automatically inform the administrator of the livestock about the birth information based on the judgment from the judgment means. Therefore, efforts of making a judgment on the measurement data and informing the administrator of the livestock about the judgment by a person having some expert knowledge or experience, such as a specialized veterinarian as an observer, can be omitted.

In the system of standby management for livestock birth according to the present invention, the livestock biological data measurement means may have at least one of a respiration rate sensor for detecting the respiration rate of the above livestock, a blood pressure sensor for detecting the blood pressure of the livestock, a hear rate sensor for detecting the heart rate of the livestock, or a body temperature sensor for detecting the body temperature of the livestock.

In the system of standby management for livestock birth according to the present invention, the livestock biological data measurement means may have a data transfer device for transmitting data. The communication line may have at least one of a telephone line or the Internet. The data relay means may have a data collector for collecting data transmitted from the above dater transfer device of the livestock biological data measurement means, a first relay device for putting the collected data on the communication line, and a second relay device for outputting a signal transmitted via the communication line. Consequently, at least one of the conventional telephone line or the Internet can be used, so that the data communication can be performed with simple and cheap structure.

In the system of standby management for livestock birth according to the present invention, the above information means may have a telephone or a facsimile device to make a simple and cheap construction to inform the above administrator about the birth information including the fact that the livestock has a birth sign or the livestock is calving and the information to specify the livestock.

Furthermore, the system of standby management for livestock birth according to the present invention may have a plurality of sets in which each set includes: the livestock biological data measurement means for measuring changes in physical conditions of the livestock due to the birth and outputting them as measurement data; the data relay means for putting the measurement data from the livestock biological data measurement means on a communication line; and the information means for informing the livestock administrator when it is judged that the livestock has a birth sign or the livestock is calving. Thus, the sets of the livestock biological data measurement means, the data relay means and the information means can be arranged, for example, on every stock farmer breeding livestock and a data display means (e.g., a data display device) is placed on a livestock management center away from those stock farmers, so that the data of physical conditions of the respective livestock having birth signs or the like of a number of stock farmers can be extensively and simultaneously observed as a unit and thus the livestock births can be intensively managed, allowing the reduction in cost to be required for birth managements of every stock farmer.

### Brief Description of the Drawings

Fig. 1 is an explanation diagram that schematically illustrates one example of the system of standby management for livestock birth;
Fig. 2 is a structural diagram that illustrates the system of standby management for livestock birth of the above example;
Fig. 3 is a structural diagram that illustrates another example of the system of standby management for livestock birth of the present invention; and
Fig. 4 is an explanation diagram that schematically illustrates still another example of the system of standby management for livestock birth of the present invention.

### Best Mode for Carrying Out the Invention

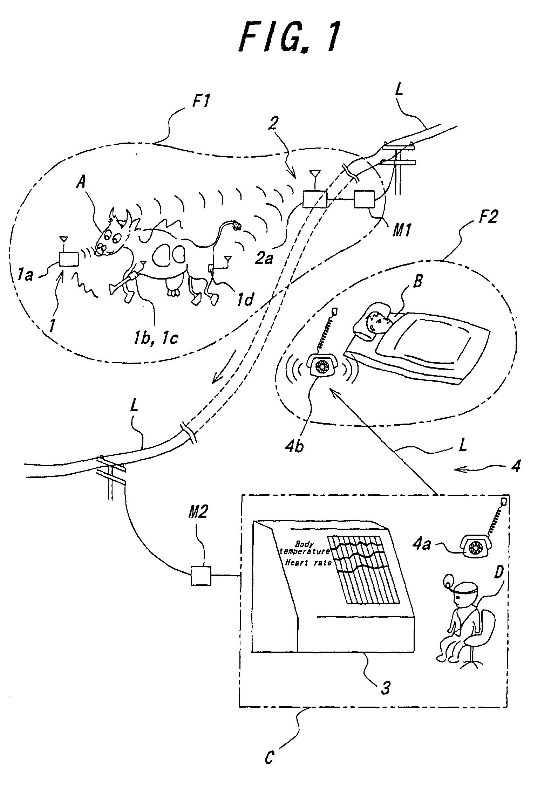

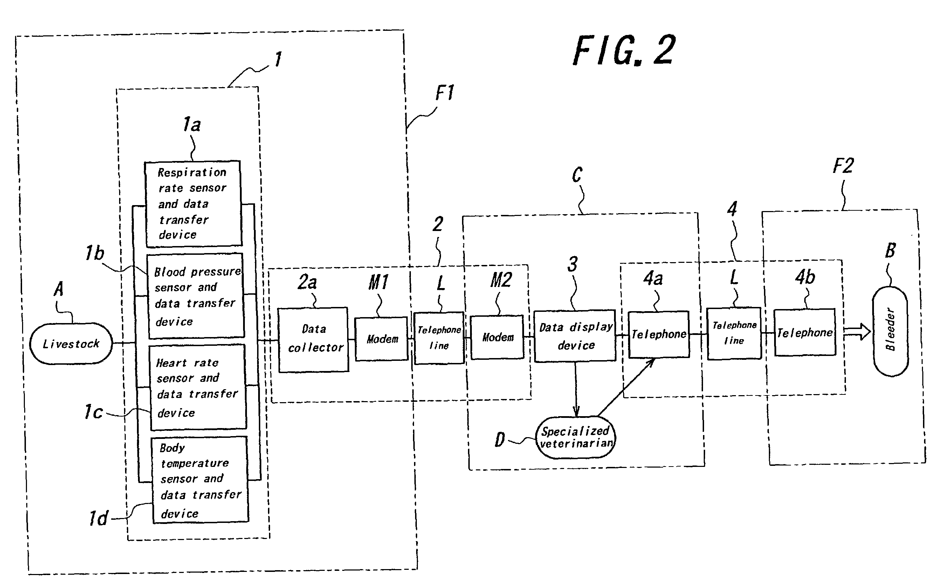

Hereinafter, an embodiment of the present invention will be described by means of examples with reference to the drawings. Fig. 1 is an explanation diagram that illustrates an example of the system of standby management for livestock birth according to the present invention, and Fig. 2 is a structural diagram that illustrates the system of standby management for livestock birth of this example. In the figures, A denotes a cow as livestock, B denotes a breeder as an administrator of the livestock A, C denotes a central management center, D denotes a specialized veterinarian, F denotes a stock farmer, F1 denotes a breeding farm of cows, F2 denotes a house of the breeder, L denotes a telephone line, M1 denotes a modem as a first relay, and M2 denotes a modem as a second relay. The telephone line L in this example is a conventional line (public line).

As shown in Fig. 1, for the cow (especially a cow being about to calve) bred at the above breeding farm F1, a biological data measurement device 1 as a livestock biological data measurement means is attached on a part of the body of the cow A. As shown in Fig. 2, the biological data measurement device 1 comprises a respiration rate sensor 1a, a blood pressure sensor 1b, a heart rate sensor 1c and a body temperature sensor 1d. Each of the sensors has a data transfer device 1a- 1d. In Fig. 1, the respiration rate sensor 1a is attached on a posterior portion near the face (nose) of the cow A (e.g., a part of the body of the cow A or in a range that allows to collect a breath of the cow A, such as a post of a low barn), the blood sensor 1b and the heart rate sensor 1c are attached on a leg of the cow A (a left front leg in Fig. 1), and the body temperature sensor 1d is attached on hip of the cow A, respectively.

Also, a data transfer device 2 as a data relay means comprises: a data collector 2a for collecting the measurement data sent from the biological data measurement device 1; a telephone line L; a modem M1 for putting the measurement data collected by the data collector 2a on the telephone line L; and a modem M2 for outputting a signal sent via the telephone line L on a data display device 3 (e.g., a personal computer) as a data display means. In addition, an information means 4 comprises: a telephone 4a placed in the central management center C by which the specialized veterinarian D informs the breeder B; and a telephone 4b placed in the house of the breeder B for receiving a signal from the telephone 4a through the telephone line L.

Furthermore, each component of the system is separately placed on the breeding farm F1 of the cow being bred at the bleeding farm F, the central management center C away from the breeding farm F1, and the house F2 of the breeder B who keeps the cow A. The biological data measurement device 1 and both the collector 2a and the modem M1 in the data transfer device 2 are placed in the site of the breeding farm F1; the modem M2 in the data transfer device 2, the data display device 3 and the telephone 4a are placed in the central management center C; and the telephone 4b is placed in the house F2, respectively. The data collector 2a may be placed at a location where the measurement data transferred from the above data transfer devices 1a- 1d can be collected. In this example, the data collector 2a is installed in an electric pole in the neighborhood of the bleeding farm F1 in Fig. 1, but it may be also installed inside the house of the breeder B such as the administrator.

When the data collector 2a is installed inside the house, it may have a receiver for receiving data from the data transfer devices 1 a- I d by means of wireless communication, a personal computer having a function of displaying the data and a warning device (e.g., a speaker) connected to the personal computer. In this way, when the specialized veterinarian detects the birth sign, the personal computer as the data display means 3 may be contrarily connected to a personal computer of the data collector 2a through the modems M1, M2 and the telephone line L to allow the personal computer of the data collector 2a to output an alarm indicating the birth from the warning device. Subsequently, the personal computer of the data collector 2a displays the livestock data to inform the bleeder B of which livestock has the birth sign or the like.

As shown in Figs. 1 and 2, in the system of standby management for livestock birth of the example, each of the measurement data (respiration rate, blood pressure, heart rate and pulse rate) of the cow A measured with each of the sensors 1a, 1b, 1c and 1d attached on the cow A can be output from the data transfer device 1a- 1d in each sensor to the modem M1 through the data collector 2a. After receiving such data, the modem M1 coverts the data into an analog signal and put the analog signal on the telephone line L. Consequently, as shown as an arrow in Fig. 1, the data can be transmitted to the modem M2. Subsequently, the modem M2 coverts the analog signal received via the telephone line L into a digital signal for the data display device 3, followed by outputting it to the data display device 3. Furthermore, based on the measurement data, the data display device 3 displays the data in such a manner that the changes in physical conditions of the cow A due to the birth can be easily judged by means of items of respiration rate, heart rate, blood pressure and body temperature in addition to their corresponding measured values and line graphs. In this case, although the above measurements are performed continuously, the measurement data measured over a fixed time period (e.g., 10 minutes) may be stored and then intermittently transmitted between the data transfer device 2 and the data display device 3 through the telephone line L.

Then, the specialized veterinarian D as the person having some expert knowledge and experience constantly monitor the results represented on the display of the data display device 3, for example, on a rotating basis in a small group. When the specialized veterinarian D judges that the cow A has the birth sign or the cow A is calving based on the displayed results, the specialized veterinarian D calls the house F2 where the bleeder B stays with the telephone 4a placed in the central management center C and the telephone 4b rings through the telephone line L. Thus, since the specialized veterinarian D informs the bleeder B as an administrator of the cow A about the birth information including the fact that the cow A has the birth sign or the cow A is calving and the information to specify the cow A (e.g., the cow's ID number), the bleeder B can stand by for the birth by attending on the cow A, helping the birth, and so on based on the birth information.

Therefore, according to the system of standby management for livestock birth of the example, sensors 1a- 1d for measuring the changes in physical conditions of the cow A due to the birth are pre-attached on the cow A, so that the measurement data of the changes in physical conditions of the cow A due to the birth transferred from these sensors 1a- 1d are constantly displayed on the display using the data display device 3 placed on the central management center C. Thus, it eliminates the need for watching the cow A and for measuring the changes in physical conditions of the cow A due to the birth with attending on the cow A, while the prior art needs them to judge that the cow A has the birth sign or the livestock is calving. Consequently, the labor burden of the specialized veterinarian D can be lightened and the birth management costs of the administrator of the stock farmer F (especially the livestock owner) or the like can be also reduced.

Furthermore, in the stock farmer F or the like breeding many cows, the respective two or more cows A may calve at a time. Even in such a case, as described above, the specialized veterinarian D has no need to perform a measurement work in directly attendance upon the cow A, while the judgment may be made by the data displayed on the display. The central management center C, therefore, can extensively and simultaneously observe and manage the data of livestock physical conditions such as the respective birth signs of many cows A as a unit. In addition, it can be performed by a small number of the specialized veterinarian with reduced burden of each person.

In addition, since the system of standby management for livestock birth of the example uses the conventional telephone line, the data communication can be attained by a simple and cheap structure. Furthermore, the telephone 4a and the telephone 4b allow that the birth information including the fact that the cow A has the birth sign or the cow A is calving and the information to specify the cow A is informed to the livestock administrator. In addition, as the measurement data is intermittently transmitted from the data transfer device 2 to the data display device 3, the charge of call can be cheaper than that of continuous data transmission.

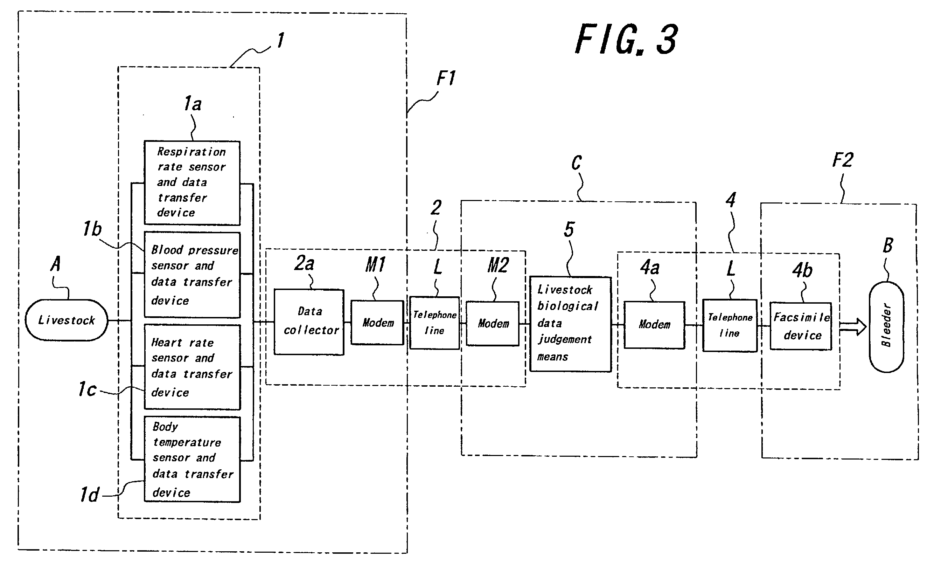

Fig. 3 is a structural diagram that illustrates another example of the system of standby management for livestock birth according to the present invention. In the figure, the reference numeral 5 denotes a livestock biological data judgment device as a livestock biological data judgment means, M3 denotes a modem for converting a digital signal output from the livestock biological data judgment device 5 into an analog signal and then putting the converted signal on the telephone line L, and 4c denotes a facsimile device to be used in stead of the telephone 4b being used in the above example. Like components as those of the aforementioned example are indicated by like numerals.

The configuration of the system of the example shown in Fig. 3 has the livestock biological data judgment device 5 instead of the data display device 3 of the aforementioned example. The livestock biological data judgment device 5 comprises a personal computer which can execute a program incorporating an algorism for performing a predetermined processing by making a judgment whether the cow A has the birth sign or whether the cow A is calving. A digital signal processed by the livestock biological data judgment device 5 is output to the modem M3 which converts a digital signal into an analog signal and put the converted signal on the telephone line L. Subsequently, the converted signal is transferred to the facsimile device 4c through the telephone line L.

Consequently, in the system of standby management for livestock birth of the example, the livestock biological data judgment device 5 makes a judgment whether the cow A has the birth sign or whether the cow A is calving and outputs the birth information as an electric signal including the fact that the cow A has the birth sign or the cow A is calving and the information to specify the cow A to the modem M3. Thus, the electric signal is entered into the facsimile machine 4c through the telephone line L, so that the facsimile device 4c can ring and the above birth information can be printed on a facsimile sheet.

Therefore, in addition to the effects of the aforementioned example, the system of standby management for livestock birth of the example can automatically make a judgment whether the cow A has the birth sign or whether the cow A is calving by the livestock biological data judgment device 5 and can automatically inform the bleeder B of the cow A about the birth information through the information means 4 based on the judgment being made by the judgment device 5. Therefore, the need for the specialized veterinarian D of making a judgment on the measurement data and informing the bleeder B of the cow A can be eliminated.

The data collector 2a may have a personal computer for allowing the display of measurement data as the aforementioned example and, instead of the facsimile device, a warning device to be executed through the telephone line L. Accordingly, the bleeder B being informed the birth sign or the like by activating the warning device can recognize which cow will calve by confirming the data display of the personal computer in the data collector 2a.

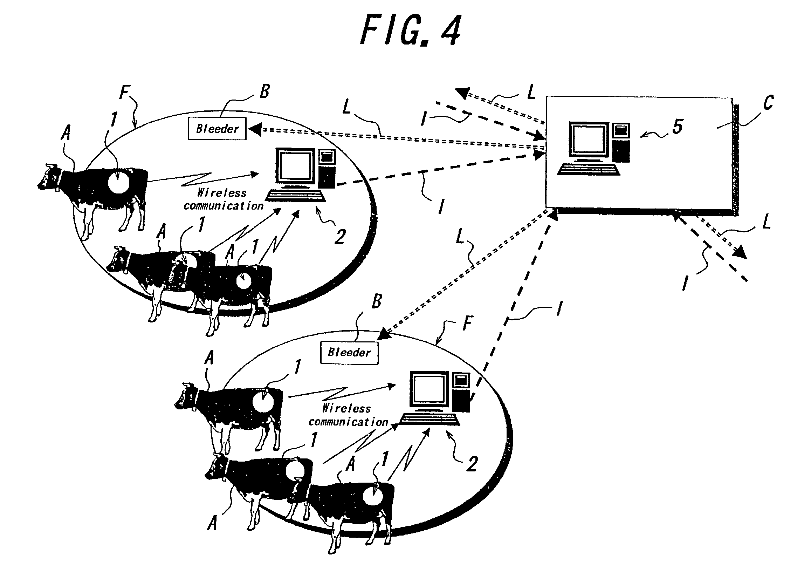

Fig. 4 is a structural diagram that illustrates a still another example of the system of standby management for livestock birth according to the present invention. In Fig. 4, like components as those of the aforementioned example are indicated by like numerals. In this example, the system comprises: a plurality of sets in which each set include a biological data measurement device 1, which is the same as that of the aforementioned example, for measuring the changes in body temperatures or the like of a plurality of springing cows A and outputting measurement data by means of wireless communication; a data transfer device 2 having a not shown receiver and a personal computer as a data relay means for receiving the measurement data and putting it on the Internet I as a communication line; and not shown telephones as a information means for informing the bleeder B as an administrator of the cow A about the birth information through the telephone line L when it is judged that the cow A has the birth sign or the cow A is calving. Each set is placed on each stock farmer F and a livestock biological data judgment device 5 comprising a personal computer that can execute a program incorporating an algorism for performing a predetermined processing by making a judgment whether the cow A has the birth sign or whether the cow A is calving. The livestock biological data judgment device 5 is connected to the Internet I.

According to the system of standby management for livestock birth of the example, in addition to the effects of the aforementioned example, it is possible to extensively and simultaneously observe the data of the birth sign of the cows A of many stock farmers F as a single unit and intensively manage the births of the cows A of many stock farmers F, so that the costs to be required for each stock farmer F to manage the birth can be reduced. In addition, for example, the stock farmers F and the central management center C can be connected to the Internet through the respective local providers. Therefore, telephone charges between the providers with respect to each other can be eliminated, and the data transmission can be performed over a wide area. Thus, for example, it is possible to manage standby of livestock birth as a single unit for stock farmers F or the like, for example, in the whole area of Hokkaido by the central management center C. In addition, the central management center C may register a domain name to directly connect to the Internet. In the central management center C, the livestock biological data judgment device 5 may be replaced with a data display means (e.g., a personal computer) and a specialized veterinarian.

As having been described with reference to the drawings, the present invention is not limited to the above examples. For example, the aforementioned two examples use the telephone line L of a public line as a communication line for transmitting the measurement data from the data transfer device 2 to the data display device 3 or to the livestock biological data judgment device 5. However, in the case where the bleeding farm F1 and the central management center C are relatively close to each other, telephone charges can be saved by making it a dedicated line. In the information means of the above examples, the communication is performed using the telephone or the facsimile device by means of a cable transmission. Alternatively, for example, the communication can be performed as a radio communication using a device such as a radio telemeter. An optical cable or the like may be used as the communication line to allow a high-speed communication. The objective livestock of the above examples is the cow A, but the present invention is not limited to it. Any livestock that requires the judgment of birth sign can be used as the object, so that a livestock such as a horse may be applicable.

## Claims

1. A system of standby management for livestock birth comprising:
one or more livestock biological data measurement means for measuring changes in physical conditions of the livestock due to the birth and outputting them as measurement data;
a data relay means for putting the measurement data from the livestock biological data measurement means on a communication line;
a data display means for receiving and displaying the measurement data transmitted via the communication line; and
an information means for informing an administrator of the livestock about birth information when it is judged that the livestock has a birth sign or the livestock is calving based on the results displayed on the data display means.

2. A system of standby management for livestock birth comprising:
one or more livestock biological data measurement means for measuring changes in physical conditions of the livestock due to the birth and outputting them as measurement data;
a data relay means for putting the measurement data from the livestock biological data measurement means on a communication line;
a livestock biological data judgment means for making a judgment whether the livestock has a birth sign or whether the livestock is calving based on the received measurement data transmitted from the communication line; and
an information means for informing an administrator of the livestock about birth information when it is judged that the livestock has the birth sign or the livestock is calving based on the results displayed on the data display means.

3. A system of standby management for livestock birth as claimed in Claim I or 2, wherein
said livestock biological data measurement means comprises at least one of a respiration rate sensor for detecting a respiration rate of said livestock, a blood pressure sensor for detecting a blood pressure of said livestock, a heart rate sensor for detecting a heart rate of said livestock, or a body temperature sensor for detecting a body temperature of said livestock.

4. A system of standby management for livestock birth as claimed in any one of Claims 1 to 3, wherein
said livestock biological data measurement means comprises a data transfer device for transferring data;
said communication line includes at least one of a telephone line or the Internet;
said data relay means comprises a data collector for collecting data transferred from the data transfer device of the livestock biological data measurement means, a first relay device for putting the collected data on the communication line, and a second relay device for outputting a signal transmitted via the communication line.

5. A system of standby management for livestock birth as claimed in any one of Claims 1 to 4, wherein
said information means comprises a telephone or a facsimile device.

6. A system of standby management for livestock birth as claimed in any one of Claims 1 to 6, further comprising a plurality of sets in which each set comprises:
said livestock biological data measurement means for measuring changes in physical conditions of the livestock due to a birth and outputting them as measurement data;
said data relay means for putting the measurement data from the livestock biological data measurement means on a communication line; and
said information means for informing the livestock administrator when it is judged that the livestock has a birth sign or the livestock is calving.
